# EUROPÄISCHE PATENTANMELDUNG

(11) **EP 1 842 486 A1**
(43) Veröffentlichungstag der Anmeldung: **10.10.2007**
(21) Anmeldenummer: 07005209.7
(22) Anmeldetag: 14.03.2007
(51) Int. Cl.: A61B 5/22, A63B 21/002, A63B 23/02, A63B 24/00

(54) **Therapieeinrichtung und Betriebsverfahren**

(30) Priorität: 14.03.2006 DE 202006004179 U
(71) Anmelder: Lupotron GmbH, 86153 Augsburg (DE)
(72) Erfinder: Wolf, Arne, Dr., 86438 Kissing (DE)
(74) Vertreter: Ernicke, Klaus Stefan

(57) **Zusammenfassung**

Die Erfindung betrifft eine Therapieeinrichtung (1) und ein Verfahren zur Diagnose und/oder zum Training der Physiologie von Probanden (2). Die Therapieeinrichtung (1) weist ein Therapiegerät (4) mit einer vom Probanden (2) mit einem Körperteil (3), insbesondere einem Oberkörperteil, zu betätigenden passiven Belastungseinrichtung (5) auf sowie eine Messeinrichtung (6) für die Probandenbetätigung und eine mit der Messeinrichtung (6) verbundene Anzeigeeinrichtung (7) im Sichtbereich des Probariden zur Signalisierung der Werte an diesen. Die Anzeigeeinrichtung (7) zeigt dem Probanden (2) die eingestellten Sollwerte und seine erreichten Istwerte.

## Beschreibung

Die Erfindung betrifft eine Therapieeinrichtung und ein Betriebsverfahren mit den Merkmalen im Oberbegriff des Verfahrens- und Vorrichtungshauptanspruchs.

Aus der Praxis sind Trainingsgeräte bekannt, mit denen von außen eine Kraft auf den Probandenkörper aufgeprägt wird. Dies kann z.B. durch Gewichte mit Umlenkung durch Hebel, durch Federn oder motorischen Antrieb, aber auch durch Schwerkraft oder Eigengewicht des Probanden oder eines Körperglieds geschehen. Derartige aktive Trainingsgeräte werfen verschiedene Probleme auf. Einerseits kann die Messung fehlerbehaftet sein, falls der Proband ausweicht und dadurch die Messung bei einer anderen als der gewünschten Auslenkung (Trainingswinkel) erfolgt. Ferner besteht eine Verletzungsgefahr durch eine Ausweichbewegung, falls der Proband die Maximalkraft nicht aufbringen kann und ausweichen will. Der Aktor des Trainingsgerätes kann bei konstanter aufgeprägter Testkraft und fehlender Gegenkraft durch den Probanden in unerwünschter Weise beschleunigen. Für Notfälle ist eine Abschaltung erforderlich, z.B. durch eine sichere Abtrennung der Energiezufuhr bei motorischen Antrieben. Eine Abschaltung bei Nutzung der Schwerkraft ist über indirektes oder aktives Eingreifen, z.B. Bremsen, möglich. Zudem ist eine Messung der physiologischen Belastung bei Schwerkraft durch Eigengewicht des Probanden oder der Körperglieds nicht möglich.

Es ist Aufgabe der vorliegenden Erfindung, eine bessere Therapietechnik aufzuzeigen.

Die Erfindung löst diese Aufgabe mit den Merkmalen im Verfahrens- und Vorrichtungshauptanspruch.

Die beanspruchte Therapieeinrichtung hat den Vorteil, dass sie sowohl zu Diagnose, wie auch zum Training des menschlichen Bewegungsapparates und insbesondere der Rumpfmuskulatur eines Probanden genutzt werden kann. Durch die passive Belastungseinrichtung muss der Proband mit einem Rumpfkörperteil gegen einen Widerstand oder eine Gegenkraft bei der Betätigung arbeiten, wobei die Parameter dieser Betätigung, z.B. Kraft, Moment und/oder Weg, zuverlässig gemessen, ausgewertet und angezeigt werden können. Unfallgefahren und unerwünschte Reaktionen wie bei den aktiven Trainingsgeräten aus dem Stand der Technik sind hierbei ausgeschlossen.

Die beanspruchte und vorzugsweise programmierbare Therapieeinrichtung ermöglicht dem Probanden über die in seinem Sichtbereich angeordnete Anzeige eine eigene Kontrolle seiner körperlichen Betätigung. Hierbei können ihm insbesondere therapeutische Vorgaben und Sollwerte neben seinen Istwerten der Betätigung angezeigt werden, so dass der Proband unmittelbar über seinen Therapie- oder Trainingserfolg informiert werden kann. Alternativ oder zusätzlich können ihm auch die Differenzwerte zwischen Soll- und Istwert angezeigt werden. Neben Einzelwerten können Wertekurven zur Anzeige gelangen, was insbesondere für komplexere Betätigungsprogramme von Vorteil ist. Der Proband kann dabei auch über die im Rahmen des Programms noch zu erledigenden Betätigungen oder Belastungen informiert werden und sich entsprechend darauf einstellen. Hierfür ist die Anzeige von Wertekurven vorteilhaft.

Die Anzeige kann in beliebig geeigneter Weise erfolgen. Günstig ist eine optische Anzeige, insbesondere mit einem graphikfähigen Bildschirm mit entsprechender Auflösung, auf der sich Kurven für Vorgabewerte und Istwerte besonders anschaulich darstellen lassen. Ergänzend oder alternativ kann eine akustische Anzeige erfolgen.

Die Einbindung der angezeigten Werte in ein Spiel oder eine andere unterhaltsame Darstellung fördert die Motivation des Probanden und den Therapieerfolg. Dabei kann es sich insbesondere um ein interaktives Spiel handeln, bei dem das Therapiegerät die Eingabeeinrichtung bzw. Spielkonsole darstellt. Der Proband und seine Betätigung können z.B. durch eine, ggf. comicartig dargestellte Spielfigur symbolisiert werden.

Das Therapiegerät kann in unterschiedlicher Weise ausgebildet sein. Es besitzt die erwähnte passive Belastungseinrichtung, die vom Probanden mit einem Körperteil, insbesondere einem Oberkörperteil zu betätigen ist. Hierbei kann der Proband ggf. über eine Führungseinrichtung in eine für das Training oder die Diagnose günstige Körperstellung gebracht und gehalten werden. Hierüber lassen sich unerwünschte Ausweichbewegungen oder Hilfsbewegungen des Probanden vermeiden. Die Führungseinrichtung zwingt den Probanden dazu, die gewünschten Körperteile in der vorgegebenen Weise zu betätigen.

Das Therapiegerät ist in der Lage, die Diagnose- bzw. Trainingsdaten und deren Auswertungen (Therapieergebnisse) bei Bedarf zu speichern und protokollieren, so dass der Therapieerfolg nachweisbar und überprüfbar ist. Insbesondere kann die Verbesserung der Physis des Probanden mit fortschreitender Zeit, auch über längere Zeiträume, dokumentiert werden.

Die Therapieeinrichtung und das Betriebsverfahren bzw. das Verfahren zur Unterstützung eines Trainings- und/oder einer Diagnose der Physiologie vom Probanden mit einer Therapieeinrichtung sind für unterschiedliche Zwecke einsetzbar. Dies betrifft zum einen medizinische und/oder physiotherapeutische Behandlungen. Andererseits lässt sich die Technik auch im normalen Fitnessbereich für das Muskeltraining, insbesondere des Oberkörpers, einsetzen. Insbesondere das Verfahren zur Unterstützung eines Trainings und/oder einer Diagnose kann auch mit entsprechend umgerüsteten oder ausgerüsteten Fitnessgeräten mit programmierbarer Belastungseinrichtung Verwendung finden. Derartige Fitnessgeräte können auch aktive Trainingsgeräte sein, wobei in diesem Fall der Erfindungsschwerpunkt auf dem Betriebs- und Unterstützungsverfahren liegt.

In der bevorzugten Ausführungsform wird das körperliche Kraftpotenzial des Probanden trainiert. Im Sichtfeld wird hierbei dem Probanden eine Kraftvorgabe Fₛₒₗₗ als statischer Wert oder als Kurve, ggf. in zeitlicher Abhängigkeit, dargestellt. Daneben wird ihm auch seine bei der Übung aufgebrachte Kraft Fist(t) angezeigt. Der Proband soll die etwaige Kraftdifferenz δF = Fₛₒₗₗ - Fᵢₛₜ jederzeit minimieren. Fₛₒₗₗ (t) ist durch einen Betreuer oder Trainer mit sinnvollen Einschränkungen wählbar. In einer praxisgerechten Ausführungsform kann es im Therapiegerät zwei fest programmierte zeitliche Vorläufe (periodische Amplitude) geben. Es kann eine Anpassung an den Probanden durch individuelle Eingabe der Vorgabe(n) Fsoll(t) geschehen. Dies kann in einem Messmodus vor dem ersten Training ermittelt und ggf. im späteren Trainingsablauf periodisch überprüft und neu ermittelt werden.

Das Therapiegerät dient insbesondere zur Kräftigung der Rumpfmuskulatur. Hierfür ist eine im wesentlichen stehende Haltung des Probanden sinnvoll. Günstig ist auch eine isometrische Kraftaufbringung auf das Polster. Diese kann auch bei geringfügiger Nachgiebigkeit des Polsters quasi isometrisch sein. Der Proband erhält die besagte Vorgabe von Fₛₒₗₗ(t), wobei seine bei der Übung aufgebrachte Kraft Fᵢₛₜ gemessen und beide Werte ständig im Blickfeld des Probanden dargestellt werden. Die Messergebnisse und ggf. auch die zugeordneten Vorgaben werden gespeichert, protokolliert und zur Dokumentation des Trainings und zur Darstellung des Trainingserfolgs und des Kraftzuwachses verwendet. Bei entsprechender Ausbildung kann das Therapiegerät mehrbenutzerfähig sein. Ein Kombigerät kann z.B. von mehreren Probanden gleichzeitig benutzt werden, wobei der apparative Aufwand durch gemeinsame Mess-, Steuer- und ggf. auch Anzeige- und Einstelltechnik reduziert werden kann.

In den Unteransprüchen sind weitere vorteilhafte Ausgestaltungen der Erfindung angegeben.

Die Erfindung ist in den Zeichnungen beispielsweise und schematisch dargestellt. Im einzelnen zeigen:
- Figur 1:: eine Therapieeinrichtung in einer schematischen Seitenansicht,
- Figur 2 und 3:: regelungstechnische Strukturbilder und
- Figur 4:: eine Anzeige mit Soll- und Istwertkurven.

Die Erfindung betrifft eine Therapieeinrichtung (1) zur Diagnose und/oder zum Training des menschlichen Bewegungsapparates, insbesondere der Rumpfmuskulatur, von Probanden (2) sowie ein Betriebsverfahren für eine solche Diagnose/Training.

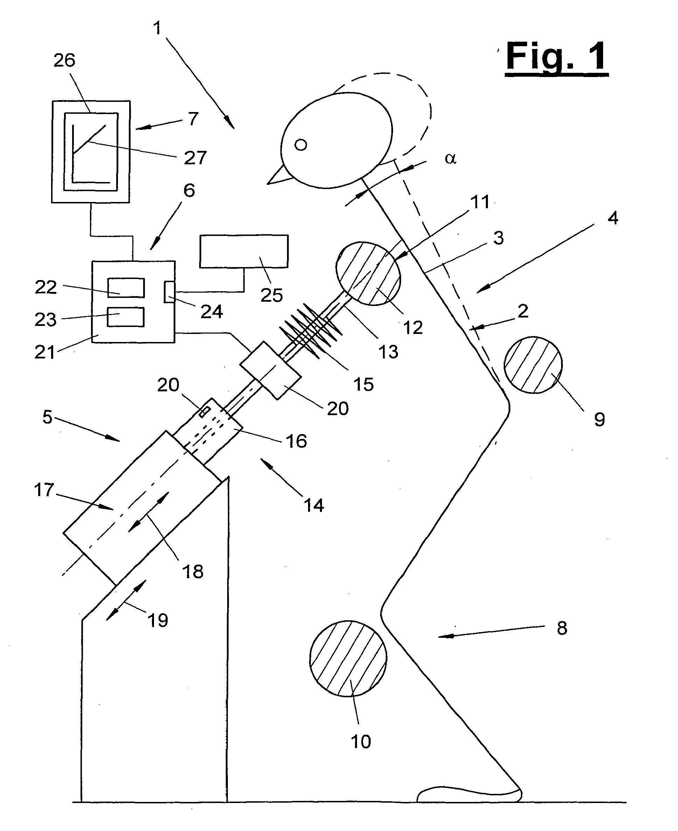

Die Therapieeinrichtung (1) ist in Figur 1 schematisch dargestellt. Sie umfasst ein Therapiegerät (4) mit einer passiven Belastungseinrichtung (5), die vom Probanden (2) mit einem Rumpfkörperteil (3), insbesondere einem Oberkörperteil oder einem Armteil, betätigt werden muss und die ggf. dieser Betätigungsbewegung nachgibt und entsprechend folgt. Bei isometrischen Übungen erfolgt kein Nachgeben.

Die Betätigungskraft ist hierbei vom Probanden (2) gegen einen festen Widerstand oder eine Gegenkraft der Belastungseinrichtung (5) aufzubringen. Hierbei werden bestimmte Körperpartien, insbesondere Muskeln oder Muskelgruppen von ein oder mehreren bestimmten Körperteilen (3) trainiert oder in ihrer Belastbarkeit diagnostiziert. Die Diagnose und/oder das Training der Muskeln oder Muskelgruppen bezieht sich auf bestimmte Auslenkungen α des betreffenden Körperteils (3), wobei diese Auslenkungen α variieren oder gleich bleiben können.

Bei den Auslenkungen α kann es sich um rotatorische oder um translatorische Bewegungen oder Kombinationen, ggf. Mehrfachkombinationen, solcher Bewegungen handeln.

In der gezeigten Ausführungsform von Figur 1 wird z.B. die Rücken- und Bauchmuskulatur des Probanden (2) diagnostiziert und/oder trainiert, wobei der Proband (2) mit der frontalen Brustpartie gegen ein Betätigungsorgan (11) der Therapieeinrichtung (1) drückt. Der zu diagnostizierende und/oder zu therapierende Körperteil ist in diesem Fall der Oberkörper. Außer der Rückenmuskulatur kann auch die Bauchmuskulatur und/oder die seitliche Rückenmuskulatur belastet werden. Bevorzugt werden Teile des Oberkörpers belastet, wobei dies z.B. auch die Arme sein können. Die Therapieeinrichtung (1) ist hierzu entsprechend anders ausgebildet und eingerichtet. Abwandlungen sind außerdem hinsichtlich anderer Muskelpartien des Oberkörpers möglich, indem z.B. eine seitliche Auslenkung des Oberkörpers verlangt wird.

Die Körperstellung des Probanden bei der Diagnose oder beim Training kann unterschiedlich sein, wobei das Therapiegerät (4) entsprechend in der Ausgestaltung differieren kann. In der gezeigten Ausführungsform nimmt der Proband eine aufrechte und stehende Position ein, wobei der Körper abgewinkelt sein kann. Alternativ kann er eine liegende oder sitzende Stellung einnehmen, wobei das Therapiegerät (4) entsprechend angepasst ist.

Das Therapiegerät (4) kann eine Führungseinrichtung (8) für den Probanden (2) beinhalten, die je nach Übungszweck unterschiedlich ausgebildet sein kann oder ggf. auch entfallen kann. In der gezeigten Ausführungsform ist z.B. eine Becken-, Hüft- oder Gesäßführung (9) und ggf. eine Knieführung (10) mit entsprechenden Polstern vorhanden. Durch die Führungseinrichtung (8) wird der Proband (2) in eine vorgegebene und für die durchzuführende Therapie günstige Körperstellung gebracht und gehalten. Dies stellt sicher, dass der Proband bei der Betätigung der passiven Belastungseinrichtung (5) genau diejenigen Körperpartien bewegt und belastet, die zur Therapie bzw. zur Diagnose und/oder zum Training vorgesehen sind. Die Führungseinrichtung (8) kann zu diesem Zweck in beliebig geeigneter Weise ausgebildet sein und andere Führungselemente an anderen Körperpartien aufweisen. Über die Führungseinrichtung (8) werden die nicht zu betätigenden Körperpartien vorzugsweise fixiert.

Die Therapieeinrichtung (1) weist eine Messeinrichtung (6) für die Probandenbetätigung und eine mit der Messeinrichtung (6) verbundene Anzeigeeinrichtung (7) im Sichtbereich des Probanden zur Signalisierung der Werte an den Probanden (2) auf. Die Messeinrichtung (6) und die Anzeigeeinrichtung (7) können Bestandteil des Therapiegeräts (4) sein. Sie können alternativ separate Komponenten darstellen.

Bei der Therapieeinrichtung (1) und dem Therapieverfahren übt der Proband (2) selbst die erforderliche Kraft aus und erhält von der Messeinrichtung (6) und der Anzeigeeinrichtung (7) eine Kontrollanzeige über seine Betätigung und über die dabei gemessenen und/oder vorgegebenen Werte oder Parameter. Dies kann z.B. die Kraft und/oder das Moment und/oder der Weg sein. Daneben sind beliebig andere, z.B. auch physiologische Parameter, wie Puls oder Blutdruck bei der Probandenbetätigung als Vorgabe möglich und erfassbar.

Von der Anzeigeeinrichtung (7) werden z.B. die mittels der Messeinrichtung (6) aufgenommenen Istwerte der Parameter der physischen Betätigung während des Trainings bzw. der Diagnose angezeigt. Zusätzlich können die Vorgabewerte oder Sollwerte angezeigt werden. Als weitere Variante ist alternativ oder zusätzlich die Anzeige der Differenzwerte oder Regelabweichungen zwischen Soll- und Istwerten möglich. Die gemessenen und/oder vorgegebenen Werte können als Einzelwerte, als gruppenweise zusammengefasste Werte oder in der bevorzugten Ausführungsform als Wertekurven (27) signalisiert und vorzugsweise optisch dargestellt werden.

Über die Anzeigeeinrichtung (7) erhält der Proband (2) eine Kontrollanzeige über seine Betätigung und über die Vorgaben sowie über etwaige Regelabweichungen. Der Proband (2) fungiert dadurch selbst als Regler und als Stellgröße für die Einstellung der gewünschten Parameter, z.B. der Kraft. Ggf. werden die physiologischen Randbedingungen, z.B. der Puls, und deren zulässige Bandbreite angezeigt. Im Falle einer Überlastung soll und wird der Proband (2) selbst die Kraft reduzieren und komplett aufgeben. Er ist dabei keiner Gegenreaktion der passiven Belastungseinrichtung (5) ausgesetzt.

Die Belastungseinrichtung (5) weist einen Widerstandserzeuger (14) für die Probandenbetätigung auf. Hierdurch wird ein physikalischer, insbesondere mechanischer, fester oder beweglicher Widerstand oder eine Gegenkraft aufgebaut, gegen die der Proband (2) bei der gewünschten physischen Betätigung arbeiten und die er ggf. überwinden muss. Hierfür weist der Widerstandserzeuger (14) ein ortsfestes oder bewegliches Betätigungsorgan (11) mit einem Anlageelement (12), z.B. dem gezeigten Brustpolster, für das zu betätigende Körperteil (3) auf.

Das bewegliche Betätigungsorgan (11) beinhaltet ein Bewegungselement, z.B. eine verschieblich gelagerte Stange (13), einen schwenkbar gelagerten Hebel (nicht dargestellt) oder dergl., welches vom Probanden (3) bei der Betätigung gegen die Wirkung der Belastungseinrichtung (5) bewegt werden muss. Der Widerstandserzeuger (14) wirkt dieser Bewegung entgegen und kann z.B. ein oder mehrere Federn (15) oder Bremselemente, z.B. eine Wirbelstrombremse, eine Reibbremse oder dgl. aufweisen.

Die Belastungseinrichtung (5) kann einen Betätigungsbegrenzer (18), z.B. einen Wegbegrenzer, einen Kraftbegrenzer oder dergl. aufweisen, welche die Betätigung des betreffenden Körperteils (3) in geeigneter Weise begrenzt und ggf. Überlastungen verhindert. In der gezeigten Ausführungsform ist der Betätigungsbegrenzer (18) ein Wegbegrenzer, der die Einfahrbewegung der Stange (13) und damit die Auslenkung α des Körperteils (3) begrenzt. Diese Begrenzung kann eine statische mechanische Einstellung in der Art von Anschlägen sein. Alternativ kann die Einstellung in einem vorher einstellbaren und begrenzbaren Winkel- oder Wegbereich variieren und insbesondere oszillieren. Dies kann z.B. über ein Exzenter im Betätigungsbegrenzer (18) erfolgen.

Ferner kann der Betätigungsbegrenzer (18) eine Dämpfung aufweisen, wobei ggf. erforderliche Wegvariationen und Wegnachführungen langsam erfolgen. Dies ermöglicht einfachere und sicherere Sicherheitsabschaltungen. Eine steuerbare Dämpfung oder Bremswirkung kann auch für den Widerstandserzeuger (14) vorhanden sein, welche die Widerstands- oder Bremskraft bei der Betätigungsbewegung und der anschließenden Rückbewegung unterschiedlich wirken lässt.

Wenn für die Diagnose und/oder das Training isometrische Übungen durchgeführt werden sollen, bei denen Muskelpartien angespannt werden, kann der Widerstandserzeuger (14) einen festen Anschlag (16) oder dergl. aufweisen, welches selbst das Anlageelement (12) bildet oder mit diesem durch die besagte Stange (13) oder dergl. fest verbunden ist.

Der Widerstandserzeuger (14) und/oder der Betätigungsbegrenzer (18) können einstellbar und verstellbar sein. Sie können hierfür insbesondere eine programmierbare Stelleinrichtung (17) aufweisen. Hierüber ist es möglich, mittels einer entsprechenden Programmierung den Widerstand bzw. die Gegenkraft des Widerstandserzeugers in Abhängigkeit von der Zeit und/oder vom Weg oder von anderen Parametern einzustellen und zu verändern. Insbesondere kann hierüber ein Belastungs- oder Gegenkraftprogramm vorgegegeben werden. Auch der Betätigungsbegrenzer lässt sich entsprechend unterschiedlich einstellen oder programmieren, so dass die vorgegebenen Auslenkungen α des Probanden (2) in der Vorgabe veränderbar sind.

Das Therapiegerät (4) kann ferner eine Anpasseinrichtung (19) für die Gerätekomponenten zur Einstellung auf unterschiedliche Körpermaße und unterschiedliche Probanden (2) aufweisen. Hierüber lassen sich die Führungseinrichtung (8) und die Belastungseinrichtung (5) entsprechend einstellen und anpassen. In der gezeigten Ausführungsform ist z.B. eine ortsfeste Konsole mit einer schrägen Linearführung vorgesehen, auf der das Gehäuse mit dem Betätigungsbegrenzer (18) und dem Betätigungsorgan (11) sowie dem Widerstandserzeuger (14) axial verstellbar gelagert und an der gewünschten Position arretierbar ist. Hierüber kann das Brustpolster (12) in der Höhe und in der Auskragung an den Körperteil (3) und die gewünsche Angriffsstelle angepasst werden. Das Einschieben der Stange (13) erfolgt z.B. gegen die Rückstellkraft von ein oder mehreren Federn (15) parallel zur schrägen Konsolenführung. An Stelle der Linearführung kann in einer nicht dargestellten Variante eine Schwenkführung für einen Hebel mit entsprechenden Einstellmöglichkeiten vorhanden sein.

Die Messeinrichtung (6) weist in der erwähnten Weise mindestens ein Messgerät (20) für ein oder mehrere physische Betätigungsparameter auf, insbesondere für die vom Probanden (2) bei der Betätigung aufzubringende Kraft, das Moment und/oder den Weg. Die physischen Betätigungs-Parameter können direkt oder indirekt gemessen werden. Z.B. kann eine Kraftmessung über eine Wegmessung in Verbindung mit einer Feder (15) oder einem Bremselement bei gegebener Federkonstante erfolgen. Bei einem festen Anschlag (16) kann eine Kraftmessung über Druckmessdosen, Dehnungsmessstreifen oder dgl. erfolgen.

Das Messgerät (20) ist in der gezeigten Ausführungsform ein Kraftmesser, der auf oder am Betätigungsorgan (11) angeordnet ist. Ein oder mehrere Messgeräte (20) können alternativ extern angeordnet sein, wobei z.B. ein optisch oder mechanisch messendes Gerät die Auslenkung α des Körperteils (3) aufnimmt.

Die Messeinrichtung (6) kann ferner ein oder mehrere Einrichtungen zur Aufnahme physiologischer Werte des Patienten aufweisen. Dies kann z.B. ein Pulsmesser, ein Blutdruckmesser, ein Messgerät für die Atemfrequenz oder dergl. sein. Diese Komponenten der Messeinrichtung (6) sind der Übersichtlichkeit halber nicht dargestellt. Auf sie kann je nach Ausbildung des Therapiegeräts (4) auch verzichtet werden.

Bei der Diagnose wird z.B. die Auslenkung α des Körperteils (3) in geeigneter Weise gemessen. Ferner können zusätzlich oder alternativ die tatsächliche Kraft und insbesondere die Maximalkraft gemessen werden, die der Proband (2) ausübt oder ausüben kann. Ferner kann eine Zeitmessung erfolgen. Insbesondere lässt sich hierbei die Maximal-Belastungsdauer messen. Dies ist die Zeit, die der Proband (2) ein bestimmtes Belastungsprofil halten kann.

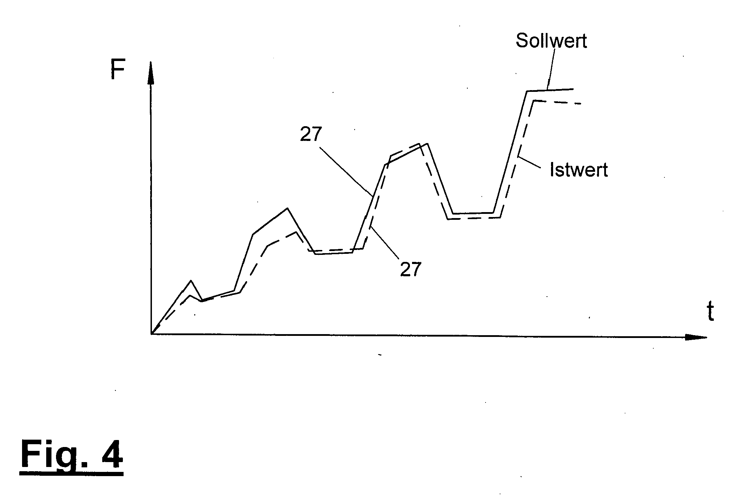

Dem Probanden wird mindestens eine Übung vorgegeben, wobei er z.B. eine Vorgabe oder ein sog. Belastungsprofil hinsichtlich Kraft und/oder Auslenkung α und Zeit bzw. Takt erhält. Ein solches Belastungsprofil ist z.B. in Figur 4 dargestellt, wobei die Gegenkraft F der passiven Belastungseinrichtung (5) über der Zeit in einem Diagramm als Wertekurven (27) aufgetragen ist. Mit durchgezogenen Strichen ist hierbei der Sollwert-Verlauf dargestellt. Der Istwert-Kurvenverlauf ist gestrichelt dargestellt. Wie Figur 4 verdeutlicht, kann in einem Belastungsprofil die Sollkraft F oszillierend und insbesondere trapezförmig langsam ansteigend vorgegeben sein. Hierbei kann zusätzlich eine mechanische Variation des mechanischen Anschlags bzw. der Wegbegrenzung erfolgen, was ebenfalls im Diagramm (nicht dargestellt) oder separat dargestellt werden kann. Über derartige von der Anzeigeeinrichtung (7) übermittelte und überlagerte Wertekurven (27) wird dem Probanden zugleich die Regelabweichung vermittelt.

Die Messeinrichtung (6) beinhaltet eine Auswerteeinrichtung (19), die vorzugsweise mit einem Sollwert-Speicher (23) ausgerüstet ist, der z.B. als statischer Werte-Speicher oder als Programmspeicher ausgebildet sein kann. Als Sollwerte können hier ein oder mehrere Werte für die messbaren oder ermittelbaren physiologischen Betätigungs-Parameter hinterlegt sein. Dies Sollwerte können anderen Werten, z.B. der Zeit, abhängen, so dass eine Sollwert-Kurve, z.B. die in Figur 4 gezeigten Kraftkurven über der Zeit hinterlegt sind. Alternativ kann z.B. eine Sollwert-Kurve die Gegenkraft F über der Auslenkung α wiedergeben.

Die Auswerteeinrichtung (19) kann ferner einen Vergleicher (22) aufweisen, der beliebig ausgestaltet sein kann. In der gezeigten Ausführungsform handelt es sich um eine Recheneinheit mit einem Mikroprozessor und einem entsprechenden Rechenprogramm.

Die Messeinrichtung (6) oder die Auswerteeinrichtung (19) können weitere Datenspeicher, z.B. für die Istwerte, für die beim Vergleich von Soll- und Istwerten gewonnenen Differenzwerte oder dergl. andere Daten aufweisen (nicht dargestellt). Insbesondere können in diesen Datenspeichern die Messergebnisse gespeichert und protokolliert werden.

Die Messeinrichtung (6) weist mindestens eine Schnittstelle (24) für die externe Kommunikation und/oder eine Eingabeeinrichtung (25), z.B. eine Tastatur, einen Kartenleser oder dergl. auf. Die Schnittstelle (24) kann z.B. eine Datenschnittstelle für den Anschluss einer Datenfernübertragung per Kabel oder kabellos, z.B. per Funk, Infrarot oder dergl. sein. An die Messeinrichtung (6) ist über eine entsprechende Schnittstelle auch die Anzeigeeinrichtung (7) angeschlossen.

Die Anzeigeeinrichtung (7) kann in unterschiedlicher Weise ausgebildet sein. Sie kann z.B. als optische Anzeige ausgebildet sein, die z.B. einen graphikfähigen Bildschirm (26), z.B. einen Flachbildschirm mit farbiger TFT- oder LCD-Anzeige oder ein anderes Display mit geeigneter Pixelmatrix und genügend hoher Auflösung, z.B. 1024 x 768 dpi, aufweist. Die optische Anzeige kann auch als Zeigerinstrument, Lauflichtbalken etc. ausgebildet sein. In vereinfachter Form kann die Anzeige ein oder mehrere Signallampen beinhalten, die Regelabweichungen von Soll- und Istwerten durch Blinken oder dergl. durch einzelnes oder reihenweises Aufleuchten oder durch Blinken und Rhythmik signalisiert. Alternativ oder ergänzend kann eine akustische Anzeige vorhanden sein, welche die Werte selbst oder die Differenzwerte durch Lautsignale, z.B. Huptöne oder durch Sprachsignale anzeigt. Ferner kann die Anzeigeeinrichtung (7) alternativ oder zusätzlich in beliebig anderer Weise ausgebildet sein und alle anderen verfügbaren menschlichen Sinnesorgane, wie Tastsinn, Geruchssinn, Temperatursinn, Gleichgewichtssinn oder dergl. über entsprechende Anzeigeelemente ansprechen.

Die Anzeigeeinrichtung (7) ermöglicht es ferner, die Werteanzeige und das Belastungsprofil in eine motivationsfördernde oder unterhaltsame mediale Umgebung einzubetten. Dies kann im einfachen Fall eine unterhaltsame Hintergrunddarstellung für die Präsentation der Soll-, Ist- und ggf. Vergleichswerte sein. Diese Hintergrunddarstellung kann statisch sein.

Ferner kann die unterhaltsame mediale Umgebung für die Wertedarstellung dynamisch und z.B. ein Spiel, insbesondere ein Videospiel, alternativ ein Hörspiel, sein. Hierbei kann es sich insbesondere um ein interaktives Spiel handeln, bei dem das Therapiegerät (4) mit seiner Belastungs-, Mess- und Auswerteeinrichtung (5,6,19) die Eingabeeinrichtung bzw. die Spielekonsole darstellt. Die Soll-, Ist- und ggf. Vergleichswerte werden dabei in einem vorprogrammierten Spieleablauf eingebettet und verarbeitet. Der Proband (2) und seine Betätigung können z.B. durch eine ggf. comicartig dargestellte Spielfigur und deren Handlungen symbolisiert werden. Die vom Proband (2) vorzunehmende(n) Übung(en) kann/können in einer programmierten Spieleumgebung dargestellt und symbolisiert werden. Eine mehrteilige Übung kann in einem dynamischen Spielablauf mit verschiedenen Szenen verpackt werden.

Dies erhöht die Motivation und reduziert die Monotonie. Regelabweichungen zwischen Soll- und Istwerten können z.B. im Rahmen eines Spiels mit Belohnungen, z.B. durch Punktegewinn, dargestellt und motiviert werden. Die vom Probanden (2) vorzunehmenden Betätigungen können von spielerisch dargestellten Hindernissen oder Aufgaben vorgestellt sein, indem z.B. eine den Probanden symbolisierende Spielfigur ein Hindernis wegdrücken muss, was eine analoge Darstellung zu der vom Probanden (2) beim Zurückschieben des Betätigungsorgans (11) vorzunehmenden Übung ist.

Der Proband (2) kann für sein Training oder für die Diagnose ein oder mehrere Übungen vorgegeben erhalten. Dies kann z.B. ein ggf. mehrere Trainingsgeräte (4) übergreifender Trainingszyklus sein. Diese Übungsvorgaben können ebenfalls in ein optisch und/oder akustisch angezeigtes Spiel oder in eine andere ideale Umgebung eingebettet sein. Der Proband (2) erhält dabei mediale Anweisungen für den Ablauf und die Durchführung seiner Übungen. Die medialen Anweisungen und Vorgaben können sich mit der Zeit, z.B. bei ansteigenden Trainingserfolgen, verändern. Ein solches Trainingsprogramm kann z.B. in der Art eines von Spielekonsolen her bekannten Jump-and-Run-Spiels oder eines anderen Videospiels vorliegen, wobei das Spiel verschiedene Schwierigkeitsstufen mit differierenden Aufgaben und optischen Hintergründen aufweist. Wenn der Proband das vorgegebene Level erfüllt hat, kann er in die nächste Spiel- oder Schwierigkeitsstufe aufsteigen.

Zur Identifizierung und Individualisierung der Probanden (2) hat das Therapiegerät (4) geeignete Schnittstellen, z.B. eine Tastatur, einen Kartenleser oder dergl.. Die einzelnen Therapiegeräte (4) können mit ihren Steuerungen und Auswerteeinrichtungen bzw. Anzeigen (7) in einem Netzwerk mit Datenaustausch und ggf. einer zentralen Steuerung mit Programm- und Datenspeicher verbunden sein. Die Datenverarbeitungs- und Speicherkapazität an den einzelnen Trainingsgeräten (4) können hierdurch entlastet sein. Ein solches Netzwerk mit zentraler Datenverarbeitung und Speicherung empfiehlt sich insbesondere bei Einsatz der vorgenannten medialen Umgebungen. Ein vom Probanden (2) zu absolvierendes Trainings- und/oder Diagnoseprogramm kann dadurch mehrere unterschiedliche Therapiegeräte (4) umfassen.

Die mit der Therapieeinrichtung (1) möglichen Therapien bzw. Diagnosen und/oder Trainingsmöglichkeiten sind vielfältig. Z.B. kann die Maximalkraft bei bestimmten Trainingswinkeln oder Auslenkungen α und vorgegebene Belastungsprofilen bzw. Wertekurven (27) ermittelt werden. Alternativ oder zusätzlich kann die maximale Belastungsdauer des Probanden (2) bei bestimmten Auslenkungen α und Belastungsprofilen ermittelt werden. Ferner ist ein Training im Bereich bestimmter Auslenkungen α mit bestimmten Sollkräften oder Belastungsprofilen möglich. Die dem Probanden (2) gezeigten Soll- und Istwerte bzw. Regelabweichungen können vorgefiltert werden, z.B. durch eine Normierung auf Sollgröße, Überhöhung, Filterung des Zeitverhaltens, Prädiktion etc., um ein besseres Regelverhalten bei der Verfolgung der Sollbelastungsprofile durch den Probanden (2) zu erreichen. Alternativ können statt Kräften Drehmomente vorgegeben und gemessen werden. Vorgebbar sind außerdem ein Sollweg und ein Istweg, z.B. bei Verwendung einer Feder zwischen Proband (2) und Messgerät (20). Die Vorgabe von Sollkraft oder Soll-Drehmoment und Soll-Weg ist mit Kraftmessung und mit Wegmessung über Federn möglich.

In Abwandlung der gezeigten Ausführungsbeispiele können mehrere physiologische Betätigungs-Parameter und zugehörige Sollwerte vorgegeben und gemessen werden. Dies kann z.B. der Diagnose und dem Training der Koordinationsfähigkeiten verschiedener Körperpartien, insbesondere Muskeln oder Muskelgruppen, dienen. Hierfür kann z.B. das Therapiegerät (4) eine mehrteilige Belastungseinrichtung (5) mit mehreren Betätigungsorganen (11) aufweisen, die in einer komplexen Bewegung gleichzeitig oder nacheinander betätigt werden müssen.

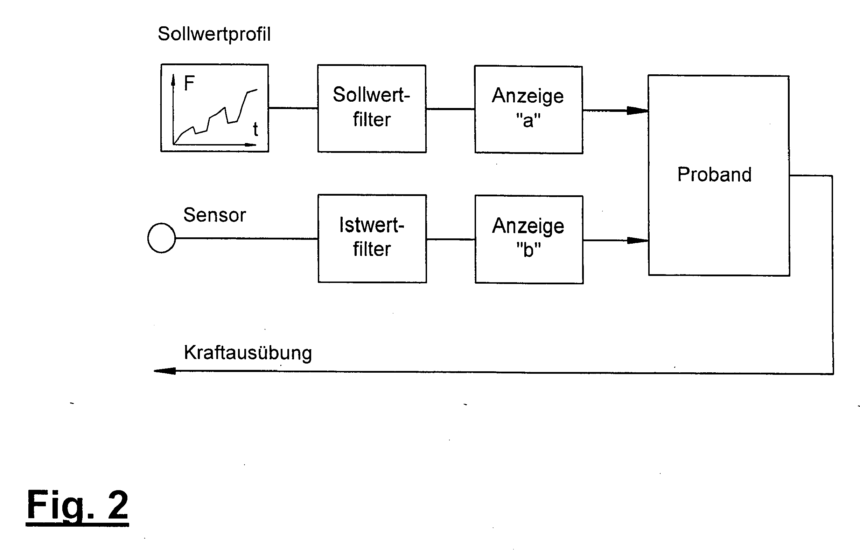

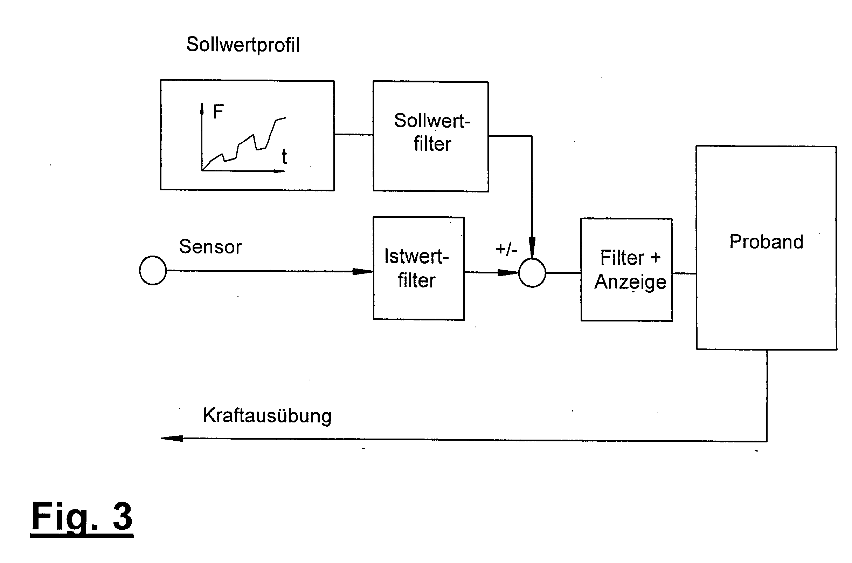

Figur 2 und 3 zeigen verschiedene Auswerte- und Darstellungsmöglichkeiten für die physischen Betätigungs-Parameter. In Figur 2 wird z.B. ein Sollwert-Profil vorgegeben und ggf. über einen Sollwert-Filter geschickt. Dies kann z.B. ein Belastungsprofil mit der Kraft über der Zeit beinhalten. Das Sollwert-Profil wird eigenständig in der Anzeigeeinrichtung (7) über eine Anzeige "a" dargestellt. Über ein oder mehrere Messgeräte (20), z.B. den in Figur 2 symbolisierten Sensor, und ggf. einen nachgeschalteten Istwert-Filter werden die Istwerte in einer zusätzlichen Anzeige "b" signalisiert. Hierbei können Soll- und Istwerte wie in der Darstellung von Figur 4 im gleichen Diagramm als Wertekurven (27) oder Wertepaare nebeneinander dargestellt sein. Alternativ können zwei getrennte Diagramme dargestellt sein. Von der Anzeige werden z.B. die aktuellen Soll- und Istwerte signalisiert. Zusätzlich können die vergangenen Werte in einer Verlaufsdarstellung signalisiert werden. Ferner können die zukünftigen Vorgabewerte angezeigt werden.

In der Variante von Figur 3 werden Soll- und Istwerte nach einem evtl. Durchlaufen von Soll- und Istwertfiltern auf einen Vergleicher gegeben, der dem Probanden über die Anzeigeeinrichtung (7) nur die Regelabweichungen bzw. die Differenzwerte signalisiert. In beiden Fällen reagiert der Proband auf die Anzeigen durch eine entsprechende und ggf. korrigierte Betätigung, insbesondere Kraftausübung.

Abwandlungen der gezeigten und beschriebenen Ausführungsformen sind in verschiedener Weise möglich. Die betrifft die Konzeption und technische Ausgestaltung des Therapiegeräts (4) und seiner Komponenten sowie der Betriebsverfahren zur Erzielung und Unterstützung der physiologischen Therapien. Variabel sind ferner die weiteren Komponenten der Therapieeinrichtung (1), insbesondere die Messeinrichtung (6) und die Anzeigeeinrichtung (7).

### BEZUGSZEICHENLISTE

- 1: Therapieeinrichtung
- 2: Proband
- 3: Körperteil, Oberkörper
- 4: Therapiegerät, Trainingsgerät
- 5: Belastungseinrichtung
- 6: Messeinrichtung
- 7: Anzeigeeinrichtung
- 8: Führungseinrichtung
- 9: Beckenführung, Beckenpolster
- 10: Knieführung, Kniepolster
- 11: Betätigungsorgan
- 12: Anlageelement, Brustpolster
- 13: Stange, Hebel
- 14: Widerstandserzeuger, Gegenkrafterzeuger
- 15: Feder
- 16: Festanschlag (Isometrie)
- 17: Stelleinrichtung
- 18: Betätigungsbegrenzer, Wegbegrenzer, Oszillator
- 19: Anpasseinrichtung
- 20: Messgerät, Kraftmesser
- 21: Auswerteeinrichtung
- 22: Vergleicher, Recheneinheit
- 23: Sollwertspeicher, Programmspeicher
- 24: Schnittstelle
- 25: Eingabeeinrichtung
- 26: Bildschirm
- 27: Wertekurve

- α: Auslenkung, Traningswinkel

## Patentansprüche

1. Therapieeinrichtung zur Diagnose und/oder zum Training der Physiologie von Probanden (2), **dadurch gekennzeichnet, dass** die Therapieeinrichtung (1) ein Therapiegerät (4) mit einer vom Probanden (2) mit einem Körperteil (3), insbesondere einem Oberkörperteil, zu betätigenden passiven Belastungseinrichtung (5), eine Messeinrichtung (6) für die Probandenbetätigung und eine mit der Messeinrichtung (6) verbundene Anzeigeeinrichtung (7) zur Signalisierung der Werte an den Probanden (2) aufweist.

2. Therapieeinrichtung nach Anspruch 1, **dadurch gekennzeichnet, dass** die Belastungseinrichtung (5) einen Widerstandserzeuger (14) für die Probandenbetätigung aufweist.

3. Therapieeinrichtung nach Anspruch 1 oder 2, **dadurch gekennzeichnet, dass** der Widerstandserzeuger (14) ein ortsfestes oder bewegliches Betätigungsorgan (11) mit einem Anlageelement (12) für das Körperteil (3) aufweist.

4. Therapieeinrichtung nach Anspruch 1, 2 oder 3, **dadurch gekennzeichnet, dass** der Widerstandserzeuger (14) ein oder mehrere feste Anschläge (16), Federn (15) oder Bremselemente aufweist.

5. Therapieeinrichtung nach einem der vorhergehenden Ansprüche, **dadurch gekennzeichnet, dass** die Belastungseinrichtung (5) einen Betätigungsbegrenzer (18), insbesondere einen Wegbegrenzer, aufweist.

6. Therapieeinrichtung nach einem der vorhergehenden Ansprüche, **dadurch gekennzeichnet, dass** die Belastungseinrichtung (5) eine Dämpfung für ein unterschiedliches Verhalten in Betätigungsrichtung und in Gegenrichtung aufweist.

7. Therapieeinrichtung nach einem der vorhergehenden Ansprüche, **dadurch gekennzeichnet, dass** der Widerstandserzeuger (14) und/oder der Betätigungsbegrenzer (18) einstellbar sind.

8. Therapieeinrichtung nach einem der vorhergehenden Ansprüche, **dadurch gekennzeichnet, dass** die Belastungseinrichtung (5) eine programmierbare Stelleinrichtung (17) für den Widerstandserzeuger (14) und/oder den Betätigungsbegrenzer (18) aufweist.

9. Therapieeinrichtung nach einem der vorhergehenden Ansprüche, **dadurch gekennzeichnet, dass** das Therapiegerät (4) eine Führungseinrichtung (8) für den Probandenkörper und die einzunehmende Körperstellung aufweist.

10. Therapieeinrichtung nach einem der vorhergehenden Ansprüche, **dadurch gekennzeichnet, dass** das Therapiegerät (4) eine Anpasseinrichtung (19) für die Gerätekomponenten zur Einstellung auf unterschiedliche Körpermaße aufweist.

11. Therapieeinrichtung nach einem der vorhergehenden Ansprüche, **dadurch gekennzeichnet, dass** die Messeinrichtung (6) mindestens ein Messgerät (20) für die physischen Betätigungs-Parameter, insbesondere Kraft, Moment und/oder Weg, aufweist.

12. Therapieeinrichtung nach einem der vorhergehenden Ansprüche, **dadurch gekennzeichnet, dass** die Messeinrichtung (6) mindestens ein Messgerät (20) für die physiologischen Werte des Probanden, insbesondere Puls und/oder Blutdruck, aufweist.

13. Therapieeinrichtung nach einem der vorhergehenden Ansprüche, **dadurch gekennzeichnet, dass** die Messeinrichtung (6) eine Auswerteeinrichtung (19) mit einem Sollwertspeicher (23) aufweist.

14. Therapieeinrichtung nach einem der vorhergehenden Ansprüche, **dadurch gekennzeichnet, dass** die Therapieeinrichtung (1) insbesondere hinsichtlich Sollwertvorgaben und Belastungsprofile programmierbar ist.

15. Therapieeinrichtung nach einem der vorhergehenden Ansprüche, **dadurch gekennzeichnet, dass** der Sollwertspeicher (23) als Programmspeicher ausgebildet ist.

16. Therapieeinrichtung nach einem der vorhergehenden Ansprüche, **dadurch gekennzeichnet, dass** die Auswerteeinrichtung (19) einen Vergleicher (22), insbesondere eine Recheneinheit, aufweist.

17. Therapieeinrichtung nach einem der vorhergehenden Ansprüche, **dadurch gekennzeichnet, dass** die Auswerteeinrichtung (19) mindestens einen Datenspeicher für die Istwerte und/oder die Vergleichswerte der Probandenbetätigung aufweist.

18. Therapieeinrichtung nach einem der vorhergehenden Ansprüche, **dadurch gekennzeichnet, dass** die Messeinrichtung (6) mindestens eine Schnittstelle (24) und/oder eine Eingabeeinrichtung (25) aufweist.

19. Therapieeinrichtung nach einem der vorhergehenden Ansprüche, **dadurch gekennzeichnet, dass** die Messeinrichtung (6) an der Anzeigeeinrichtung (7) die Werte oder Wertekurven (27) der Istwerte und/oder Sollwerte und/oder Differenzwerte anzeigt.

20. Therapieeinrichtung nach einem der vorhergehenden Ansprüche, **dadurch gekennzeichnet, dass** die Anzeigeeinrichtung (7) als optische Anzeige ausgebildet ist.

21. Therapieeinrichtung nach einem der vorhergehenden Ansprüche, **dadurch gekennzeichnet, dass** die Anzeigeeinrichtung (7) mindestens einen Bildschirm (26) aufweist.

22. Therapieeinrichtung nach einem der vorhergehenden Ansprüche, **dadurch gekennzeichnet, dass** die Werteanzeige in eine Unterhaltungsumgebung, insbesondere ein Spiel, eingebunden ist.

23. Verfahren zur Unterstützung eines Trainings und/oder einer Diagnose der Physiologie von Probanden (2) mit einer Therapie- oder Fitnesseinrichtung (1),
**dadurch gekennzeichnet, dass** dem Probanden (2) von einer programmierbaren Therapie- oder Fitnesseinrichtung (1) über eine Anzeige (7) die Vorgaben oder Anweisungen, insbesondere Belastungsprofile, für das Training und/oder die Diagnose eingebettet in ein Spiel, insbesondere ein Videospiel, übermittelt werden.

24. Verfahren nach Anspruch 23, **dadurch**
**gekennzeichnet, dass** in einem interaktiven Spiel die Istwerte und/oder Vergleichswerte der Probandenbetätigung verarbeitet und dargestellt werden.
